(19) Europäisches Patentamt
European Patent Office
Office européen des brevets

(11) EP 2 870 956 A1

(12) **EUROPEAN PATENT APPLICATION**

(43) Date of publication:
**13.05.2015 Bulletin 2015/20**

(51) Int Cl.:
***A61K 6/00*** *(2006.01)*

(21) Application number: **13005318.4**

(22) Date of filing: **11.11.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Dentsply DeTrey GmbH**
**78467 Konstanz (DE)**

(72) Inventors:
- **Klee, Joachim E.**
**78315 Radolfzell (DE)**
- **Maier, Maximilian**
**40213 Düsseldorf (DE)**
- **Lalevée, Jacques**
**68200 Mulhouse (FR)**
- **Fouassier, Jean Pierre**
**68590 St Hippolyte (FR)**
- **Morlet-Savary, Fabrice**
**68120 Pfastatt (FR)**

(74) Representative: **Hartz, Nikolai**
**Wächtershäuser & Hartz**
**Patentanwaltspartnerschaft mbB**
**Weinstrasse 8**
**80333 München (DE)**

(54) **Dental composition**

(57) Photocurable dental composition comprising
(a) one or more radical-polymerizable monomers, and
(b) 0.1 to 7.0 percent by weight based on the total weight of radical polymerizable monomers in the composition of a compound of the following formula (I):

$$R^1R^2R^3X(CO)X'R^4R^5R^6 \qquad (I)$$

wherein
X and X' which may be the same or different, independently represent Si or Ge,
$R^1$, $R^2$, $R^3$, $R^4$, $R^5$, and $R^6$, which may be the same or different, independently represent a hydrocarbon group having 1 to 20 carbon atoms, whereby any two hydrocarbon groups of $R^1$, $R^2$, $R^3$, $R^4$, $R^5$, and $R^6$ may be bonded to each other and form together with either X, X' or the moiety X(CO)X' to which they are attached a 3 to 12- membered heterocyclic ring, whereby any of the hydrocarbon group or the heterocyclic ring may
- contain one or more keto groups other than the keto group of the X(CO)X' moiety, or
- be substituted by on or more groups selected from halogen atoms, a cyano group, an amino group or a hydroxy group, and- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group.

EP 2 870 956 A1

**Figure 1.**

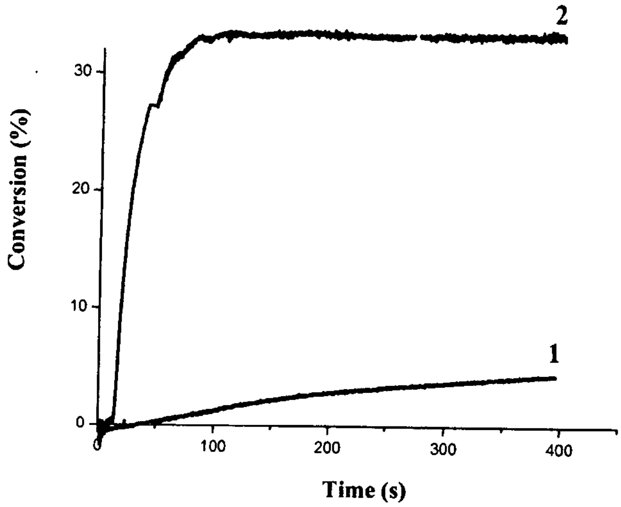

## Description

### Field of the invention

The present invention relates to a dental composition containing a specific photoinitiator compound which is useful for initiating radical polymerization of radical-polymerizable monomers contained in a photocurable dental composition. The present invention also relates to the use of the photoinitiator for the preparation of a photocurable dental composition.

A dental composition according to the present invention has increased polymerization efficiency and curing speed so that the mechanical properties of a cured dental composition may be improved.

### Background of the invention

The restoration of teeth commonly involves a light curable dental composition containing free-radically polymerizable resins. Light curing of a dental composition involves a photoinitiator system generating free radicals upon exposure to visible light. Free radicals may be typically produced by either of two pathways:

(1) the photoinitiator compound undergoes excitation by energy absorption with subsequent decomposition of the compound into one or more radicals (Norrish type I), or

(2) the photoinitiator compound undergoes excitation and the excited photoinitiator compound interacts with a second compound by either energy transfer or a redox reaction to form free radicals from any of the compounds (Norrish type II).

In order for a photoinitiator to be useful for use in a dental composition, the quantum yields indicating the conversion of light radiation to radical formation needs to be high since absorption or shielding of light by further components of the dental composition limit the amount of energy available for absorption by the photoinitiators. Accordingly, only 70 percent conversion of the polymerizable groups may be expected in a polymerization of a typical dental composition, whereby the mechanical strength of the polymerized dental composition is less than optimal and unreacted monomers may leach out from the polymerized dental composition. The leaching monomers may have detrimental effects. In order to alleviate this problem, multifuctional monomers are frequently used which are more likely to be included in the polymer network.

In addition, photoinitiators are required to have a high solubility, thermal stability, and storage stability when incorporated into a dental composition.

Finally, given that dental compositions usually contain (meth)acrylate or (meth)acrylamide monomers, free radical photocuring may be inhibited by the presence of oxygen. Oxygen inhibition is due to the rapid reaction of propagating radicals with oxygen molecules to yield peroxyl radicals which are not as reactive towards carbon-carbon unsaturated double bonds and therefore do not initiate or participate in any photopolymerization reaction. Oxygen inhibition may lead to premature chain termination and, therefore, incomplete photocuring.

Accordingly, the polymerization initiator system has a critical influence on the quality of the dental material. Camphor quinone in combination with tertiary amine is frequently used as photoinitiator system. However, the presence of amines in acrylate-containing compositions can cause yellowing in the resulting photocured composition, create undesirable odors, and soften the cured composition because of chain transfer reactions and therefore, often require the use of stabilizers. Moreover, the use of aromatic amines such as ethyl 4-dimethylamino benzoate gives rise to toxicological concerns.

Furthermore, it is desirable that the light activating the photoinitiator system has a long wavelength in order to avoid damage of soft tissue during polymerization of the dental composition in the patient's mouth. Accordingly, the photoinitiator system is required to contain a chromophoric group efficiently absorbing light of the desired wavelength in a range of from 400 to 800 nm. However, an increase of the absorption coefficient of the photoinitiator system increases the coloration of the photoinitiator system and thereby the coloration of the dental composition.

Accordingly, it is necessary that the chromophoric groups are efficiently destroyed during polymerization so that the coloration of the initiator system disappears in the polymerized dental composition. A destruction of the chromophoric groups during polymerization may also be useful in increasing the depth of cure of the dental composition since activating light is not shielded from unpolymerized layers of the dental composition by the photoinitiator system present in polymerized layers covering the unpolymerized layers.

M.-A. Tehfe et al. Macromol. Rapid Commun. 2010, 31, 473-478 disclose bis(germylketones) as type I photoinitiator systems sensitive above 500 nm. However, a dental composition or any composition comprising a particulate filler and/or solvent is not disclosed.

**Summary of the invention**

It is the problem of the present invention to provide a dental composition containing radical-polymerizable monomers which has an improved polymerization efficiency, high curing speed and high storage stability, and which does not give rise to coloration problems so that the mechanical strength of a dental composition may be improved as well as the adhesion of the polymerized dental composition to enamel and dentin. Moreover, it is the problem of the present invention to provide a use of a specific compound for the preparation of a dental composition.

The present invention provides a dental composition comprising

(a) one or more radical-polymerizable monomers,

(b) 0.1 to 7.0 percent by weight based on the total weight of radical polymerizable monomers in the composition of a compound of the following formula (I):

$$R^1R^2R^3X(CO)X'R^4R^5R^6 \qquad (I)$$

wherein

X and X' which may be the same or different, independently represent Si or Ge,

$R^1$, $R^2$, $R^3$, $R^4$, $R^5$, and $R^6$, which may be the same or different, independently represent a hydrocarbon group having 1 to 20 carbon atoms, whereby any two hydrocarbon groups of $R^1$, $R^2$, $R^3$, $R^4$, $R^5$, and $R^6$ may be bonded to each other and form together with either X, X' or the moiety X(CO)X' to which they are attached a 3 to 12-membered heterocyclic ring,

whereby any of the hydrocarbon group or the heterocyclic ring may

- contain one or more keto groups other than the keto group of the X(CO)X' moiety, or
- be substituted by on or more groups selected from halogen atoms, a cyano group, an amino group or a hydroxy group, and
- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group, and

(c) a particulate filler and/or solvent.

Moreover, the present invention provides the use of a compound of the following formula (I):

$$R^1R^2R^3Ge(CO)GeR^4R^5R^6 \qquad (I)$$

wherein X, X', $R^1$, $R^2$, $R^3$, $R^4$, $R^5$, and $R^6$, are as defined above, for the preparation of a dental composition.

The present invention is based on the recognition that a ketone compound of formula (I) according to the present invention provides improved polymerization efficiency, high curing speed and high storage stability, and does not give rise to coloration problems of a dental composition comprising radical-polymerizable monomers. Accordingly, according to the present invention a relatively large amount of the dental composition can be photocured with reduced exposure to radiation. Due to the high efficiency of the photoinitiator compound, the presence of oxygen, or oxygen inhibition, is not a serious detriment during photocuring of a dental composition according to the present invention. Finally, since the photocuring speeds are high in a dental composition of the present invention, the dental compositions may be pigmented or filled so that light penetration is limited.

**Brief description of the Figures**

Figure 1 shows photopolymerization profiles of HDDA in laminate: (1) without photoinitiator and (2) in the presence of DGK 1.5 % w/w; upon a Xenon lamp exposure filtered for $\lambda$ > 400 nm.

Figure 2 shows photopolymerization profiles of TMPTA in laminate: (1) in the presence of DGK (2 % w/w) and (2) in the presence of DGK/diphenyliodonium hexafluorophosphate (2%/2% w/w); upon a laser diode irradiation@ 473 nm (100 mW/$cm^2$).

**Detailed description of preferred embodiments**

The term "polymerization" relates to the combining by covalent bonding of a large number of smaller molecules, such as monomers, to form larger molecules, that is, macromolecules or polymers. The monomers may be combined to form only linear macromolecules or they may be combined to form three-dimensional macromolecule, commonly referred to as crosslinked polymers. In case of a higher conversion rate of the polymerizable monomer, the amount of multifunctional monomers may be reduced or the leaching problem may be alleviated.

The terms "curing" and "photocuring" mean the polymerization of functional oligomers and monomers, or even polymers, into a crosslinked polymer network. Curing is the polymerization of unsaturated monomers or oligomers in the presence of crosslinking agents.

The terms "photocurable" and "curable" refer to a dental composition that will polymerize into a crosslinked polymer network when irradiated for example with actinic radiation such as ultraviolet (UV), visible, or infrared radiation.

The term " radical-polymerizable monomers" is meant to include any unsaturated material having one or more carbon-to-carbon double bonds (ethylenically unsaturated groups) capable of undergoing polymerization. The term encompasses unsaturated monomers, oligomers, and crosslinking agents. The singular form of the term is intended to include the plural. Oligomeric and multifunctional acrylates are examples of radical-polymerizable monomers.

The term "quantum yield" is used herein to indicate the efficiency of a photochemical process. More particularly, quantum yield is a measure of the probability that a particular molecule will absorb a quantum of light during its interaction with a photon. The term expresses the number of photochemical events per photon absorbed.

"Actinic radiation" is any electromagnetic radiation that is capable of producing photochemical action and can have a wavelength of at least 150 nm and up to and including 1250 nm, and typically at least 300 nm and up to and including 750 nm.

The present invention relates to a dental composition. The dental composition may be a dental adhesive composition, a dental composite, a resin modified dental cement, and a pit and fissure sealant. The dental composition may be cured by irradiation of actinic radiation.

The dental composition contains one or more radical-polymerizable monomers. The radical-polymerizable monomers may preferably be a polymerizable N-substituted alkyl acrylic or acrylic acid amide monomer or a (meth)acrylate compound.

A polymerizable N-substituted alkyl acrylic or acrylic acid amide monomer may be preferably selected from compounds characterized by one of the following formulas:

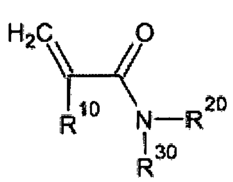

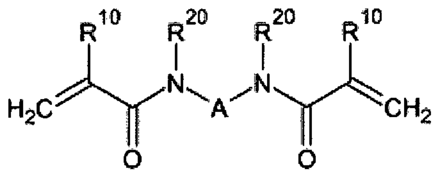

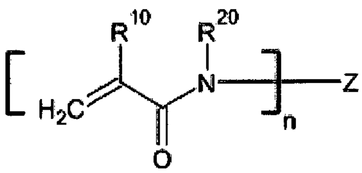

wherein $R^{10}$, $R^{20}$ and $R^{30}$ independently represent a hydrogen atom or a C1 to C8 alkyl group; A represents a divalent substituted or unsubstituted organic residue having from 1 to 10 carbon atoms, whereby said organic residue may contain from 1 to 3 oxygen and/or nitrogen atoms; Z represents a saturated at least trivalent substituted or unsubstituted C1 to C8 hydrocarbon group, a saturated at least trivalent substituted or unsubstituted cyclic C3 to C8 hydrocarbon group, and n is at least 3. Preferably, radical-polymerizable monomers include bisacrylamides such as N,N'-diethyl-1,3-bisacrylamido-propan (BADEP), 1,3-bisacrylamido-propan (BAP), 1,3-bisacrylamido-2-ethyl-propan (BAPEN), N,N'-(2E)-but-2-en-1,4-diallylbis-[(N-prop-2-en-1) amid (BAABE) and N,N-di(cyclopropyl acrylamido) propane (BCPBAP).

A (meth)acrylate compound may be selected from the group of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl

dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, and 2,2'-bis[3(4-phenoxy)-2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Preferably, the polymerizable N-substituted alkyl acrylic or acrylic acid amide monomer or (meth)acrylate compound has a molecular weight of at most 400, more preferably at most 300.

Preferably, the radical-polymerizable monomers (a) each contain one or two radical-polymerizable groups.

It is preferable that a blending ratio of the radical-polymerizable monomers to the entire dental composition is 5 to 80% by weight. More preferably, the blending ratio is 10 to 60% by weight.

According to a preferred embodiment, the dental composition of the present invention comprises a radical-polymerizable monomer having an acidic group.

Moreover, the dental composition according to the present invention comprises 0.1 to 7.0 percent by weight based on the total weight of radical polymerizable monomers in the composition of a compound of the following formula (I):

$$R^1R^2R^3X(CO)X'R^4R^5R^6 \qquad (I)$$

The compound of formula (I) may act as a type I photoinitiator whereby a single photon absorbed by the compound may generate two radicals $R^1R^2R^3X$ and $X'R^4R^5R^6$ which are able to polymerize the dental composition. Accordingly the quantum yield of the compound formula (I) may be twice as high as an equimolar amount of camphor quinone/amine initiator.

Preferably, the dental composition contains comprises less than 2.5 percent by weight, preferably 0.5 to 2.0 percent by weight based on the total weight of radical polymerizable monomers in the composition of a compound of formula (I).

In formula (I), X and X' which may be the same or different, independently represent Si or Ge.

According to a first embodiment, X and X' are the same. Accordingly, the compound of formula (I) is a bis(silyl) ketone of the following formula (II) or a bis(germyl) ketone of the following formula (III)

$$R^1R^2R^3Si(CO)SiR^4R^5R^6 \qquad (II)$$

$$R^1R^2R^3Ge(CO)GeR^4R^5R^6 \qquad (III)$$

According to a second embodiment, X is Si and X' is Ge according to the following formula (IV):

$$R^1R^2R^3Si(CO)GeR^4R^5R^6 \qquad (IV)$$

In formula (I), (II), (III), and (IV), the moieties $R^1$, $R^2$, $R^3$, $R^4$, $R^5$, and $R^6$ may be the same or different, and independently represent a hydrocarbon group having 1 to 20 carbon atoms, preferably having 2 to 8 carbon atoms, and more preferably 2 to 6 carbon atoms.

The hydrocarbon group may be and alkyl group, a cycloalkyl group, a cycloalkylalkyl group, an aralkyl group or an aryl group.

An alkyl group may be straight-chain or branched $C_{1-20}$ alkyl group, typically a $C_{1-8}$ alkyl group. Examples for

a $C_{1-6}$ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a $C_{3-20}$ cycloalkyl group. Examples of the cycloalkyl group can include those having 3 to 14 carbon atoms, for example, cyclopropyl, cyclobutyl, cyclopentyl and cyclohexyl. A cycloalkylalkyl group can include those having 4 to 20 carbon atoms.

A cycloalkylalkyl group can include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl.

An aralkyl group may be a $C_{7-20}$ aralkyl group, typically a combination of a linear or branched alkyl group having 1 to 6 carbon atoms and an aryl group having 6 to 10 carbon atoms. Specific examples of an aralkyl group are a benzyl group or a phenylethyl group.

An aryl group can include aryl groups having 6 to 10 carbon atoms. Examples of the aryl group are phenyl and naphtyl. Aryl groups may contain 1 to 3 substituents. Examples of such substituents can include halogen atoms, a cyano group, a hydroxy group, an amino group, $C_{1-6}$ alkyl groups and $C_{1-6}$ alkoxy groups. Here, illustrative of the halogen atoms can be fluorine, chlorine, bromine and iodine. The $C_{1-4}$ alkyl groups are, for example, methyl, ethyl, n-propyl, isopropyl and n-butyl. Illustrative of the $C_{1-4}$ alkoxy groups are, for example, methoxy, ethoxy and propoxy. The alkyl moieties in these substituents may be linear, branched or cyclic.

Preferably, the hydrocarbon group is an aryl group selected from a phenyl group and a naphthyl group, which groups may optionally be substituted by one to three groups selected from halogen atoms, a cyano group, an amino group, a hydroxy group, $C_{1-6}$ alkyl groups and C1-6 alkoxy groups, or wherein the hydrocarbon group is a non-aromatic hydrocarbon group selected from a straight chain or branched alkyl group, a straight chain or branched alkenyl group, or a straight chain or branched alkynyl group.

The $C_{1-8}$ alkyl group and the $C_{3-14}$ cycloalkyl group may optionally be substituted by one or more members of the group selected from a $C_{1-4}$ alkyl group, $C_{1-4}$ alkoxy group, a phenyl group, and a hydroxy group. Examples for a $C_{1-4}$ alkyl group can include linear or branched alkyl groups having 1 to 4 carbon atoms, for example, methyl, ethyl, n-propyl, isopropyl, n-butyl, isobutyl, sec-butyl, tert-butyl. Examples for an $C_{1-4}$ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

In formula (I), any two hydrocarbon groups of $R^1$, $R^3$, $R^3$, $R^4$, $R^5$, and $R^6$ may be bonded to each other and form together with either X, X' or the moiety X(CO)X' to which they are attached a 3 to 12- membered heterocyclic ring.

In formula (I), any of the hydrocarbon group or the heterocyclic ring may contain one or more keto groups other than the keto group of the X(CO)X' moiety,

Moreover, in formula (I), any of the hydrocarbon group or the heterocyclic ring may be substituted by on or more groups selected from halogen atoms, a cyano group, an amino group or a hydroxy group. Accordingly, in the hydrocarbon groups some or all hydrogen atoms are replaced by halogen atoms (e.g., fluoro, bromo, chloro), for example, halo-substituted alkyl groups such as chloromethyl, chloropropyl, bromoethyl and trifluoropropyl, and cyanoethyl.

In case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group. In case the hydrocarbon group is an alkyl group having more than one carbon atom, the alkyl group contains an alkylene Accordingly, in case the hydrocarbon group is an n-hexyl group, any of the carbon atoms of the alkylene chain excluding the terminal methyl group may be replaced by an oxygen atom, a sulfur atom, an amide group, an ester group, a urethane group or an NH group. Therefore, the following groups may be given as specific examples in case of one or more oxygen atoms:

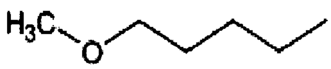

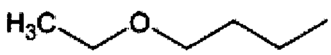

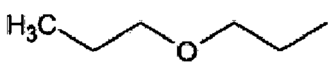

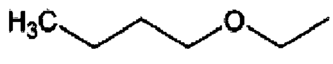

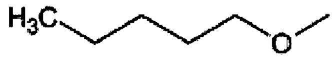

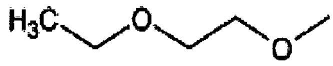

Specific examples for a compound of formula (I) include bis(triphenylgermyl)methanone, bis(triethylgermyl)methanone, and bis(trimethylgermyl)methanone.

A compound of the formula (I) may be a known compound which may be prepared according to published procedures. Alternatively, a compound of formula (I) may be prepared according to the following scheme (Johannesen R. et al, J. Chem. Soc. Perkin Trans. 1, 2000, 2677-2679).

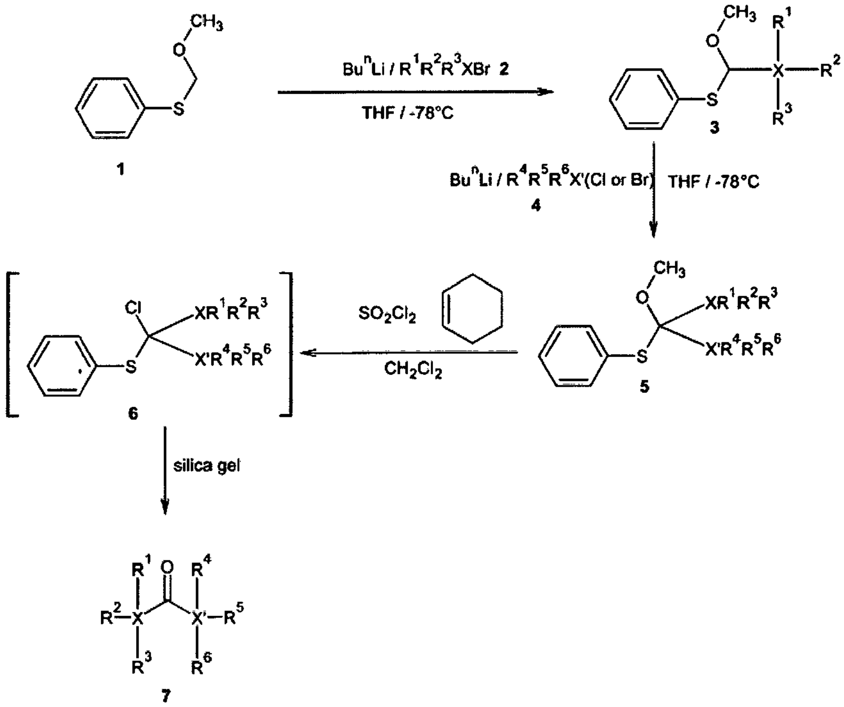

1
3
5
6
7

In the above scheme, X is preferably Ge.

According to the preparation method a compound **1** is treated with a strong base in an aprotic solvent at low temperature followed by reaction with a compound **2** for generating a compound **3.** The reaction temperature may be in the range of from - 20 to -100 °C, preferably -40 to -80 °C. The reaction time is not particularly limited and may be selected in the reange of from 1 to 24 hours, preferably 2 to 10 hours. The reaction mixture may be quenched by the addition of water and the product may be extracted into benzene, washed with water, dried ($MgSO_4$) and evaporated to give **3.** The crude product may be used in the next step without further purification.

**3** may be treated with a strong base in an aprotic solvent at low temperature followed by reaction with a compound **4** for generating a compound **5.** The reaction temperature may be in the range of from 40 to -80 °C, preferably 30 to -20 °C. The reaction time is not particularly limited and may be selected in the reange of from 1 to 24 hours, preferably 2 to 10 hours. The reaction mixture may be quenched by the addition an aqueous solution of $NH_4Cl$ (10%) and the product may be extracted into benzene, washed with water, dried ($MgSO_4$) and evaporated to give 5. The crude product may be used in the next step without further purification.

In case a symmetrical ketone is prepared, a compound **5** may be obtained in a single step by using two equivalents of **2.**

A compound **7** used in the present invention may be prepared by adding sulfuryl dichloride in dry dichloromethane dropwise to a solution of compound **5** in dry dichloromethane under nitrogen. The mixture is stirred for 30 min before a

solution of cyclohexene in dry dichloromethane is added dropwise. The reaction temperature is not particularly limited and may be selected in the range of from 40 to -20 °C, preferably 0 °C. Subsequently, the solvent is removed and the residue is eluted through a silica gel column under inert atmosphere, preferably argon gas, using first degassed pentane as eluent, then a degassed mixture of pentane-diethyl ether 20:1 and a compound for formula (I) may be isolated as a compound of formula (I) of the present invention.

Alternatively a symmetrical bis(germyl)ketone may also be prepared by adding to a trisubstituted germanium hydride such as triphenylgermanium hydride at low temperature such as at -23 °C, n-BuLi and subsequently dropwise ethyl formate (preferably freshly distilled over $CaH_2$) Subsequently, the reaction mixture may be allowed to warm at room temperature over before being quenched with aqueous hydrochloric acid. The organic phase is separated and the aqueous phase is extracted three times with diethyl ether. The combined organic phases are dried over sodium sulfate, filtered and concentrated to give the corresponding bis(germyl) methanol product. To a solution of bis(germyl) methanol in dry diethyl ether, successively dicyclohexylcarbodiimide and pyridinium trifluoroacetate were added. The flask is then carefully protected from light and dry DMSO is added dropwise. The stirring is continued overnight. The supernatant liquid may then then be withdrawn via syringe and filtered through a 0.2 μm syringe filter. After the filtrate is evaporated under vacuum in the absence of light, the desired bis(germyl)methanone is obtained.

Preferably, a compound of formula (I) has an absorption maximum in the range of from 400 nm to 900 nm. Accordingly, polymerization may be carried out by using blue or green LED light, preferably in the range of from 460 to 530 nm.

The dental composition may contain an additional initiator which is selected from a Norrish type I initiator and a Norrish type II initiator.

A Norrish type I initator provides free radical intermediates by a photochemical cleavage or homolysis an initiator molecule. Examples of suitable additional initiators include iodonium salts, peroxides or diazo compounds. Suitable free-radical polymerization initiators may be selected from organic peroxides such as benzoylperoxide, methylethylketone peroxide, acetone peroxide and tert-butyl hydroperoxide, azo compounds such as azobisisobutyronitrile and 1,1'azo-bis(cyclohexanecarbonitrile).

A Norrish type II initator provides free radical intermediates by the photochemical abstraction. Suitable Norrish type II initiators may be a combination of a sensitizing material and a reducing material. The sensitizing material could be camphorquinone, benzyl, diacetyl, benzyl dimethyl ketal, benzyl diethyl ketal, benzyl di(2-methoxyethyl) ketal, 4,4'-dimethylbenzyl-dimethyl ketal, anthraquinone, 1-chloroanthraquinone, 2-chloroarithraquinone, 1,2-benzanthraquinone, 1-hydroxyanthraquinone, 1-methytanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluorothioxanthone, thioxanthone-10,10-dioxide, thioxanthone-10-oxide, benzoin methyl ether, benzoin ethyl ether, isopropyl ether, benzoin isobutyl ether, benzophenone, bis(4-dimethylaminophenyl) ketone, 4,4'-bisdiethylaminobenzophenone, and a compound including an azide group. These sensitizing materials can be used independently or by mixing one or more of them.

A tertiary amine may be used as a reducing material. Examples of the tertiary amine include triethanolamine, methyl 4-dimethylaminobenzoate, ethyl 4-dimethylaminobenzoate, dimethylaminoethyl methacrylate, or isoamyl 4-dimethylaminobenzoate.

In addition to these reducing materials, an organic metal compound, or a sulfinic acid derivative, can be used as a reducing material.

Preferably, the additional initiator is selected from camphor quinone/amine, iodonium salts, sulfonium salts and phosphonium salts. Preferably, the additional initiator is a compound of the following formula:

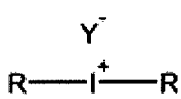
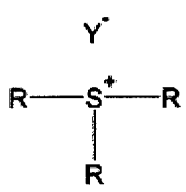
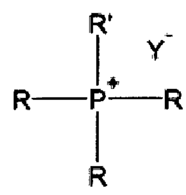

wherein the R which may be the same or different represent an aryl group which may be substituted, R' represents a hydrocarbon group and $Y^-$ is an anion selected from hexafluoroantimonate, trifluoromethylsulfate, hexafluorophosphate, tetrafluoroborate, hexafluoroarsenate, and tetraphenylborate.

Preferably, R is a phenyl group which may be substituted with 1 to 3 substituents selected from halogen atoms, a cyano group, a hydroxy group, an amino group, $C_{1-6}$ alkyl groups and $C_{1-6}$ alkoxy groups.

Preferably, R' is a linear, branched or cyclic alkyl group having 1 to 6 carbon atoms, which may be substituted with 1 to 3 groups selected from halogen atoms, a cyano group, a hydroxy group, an amino group, $C_{1-6}$ alkyl groups and $C_{1-6}$ alkoxy groups.

The additional initiator may be used in a ratio of an amount of the compound of formula (I) to an amount of the additional initiator of

$$0.1 \leq (\text{mass of compound of formula (I)/additional initiator}) \leq 10.$$

The use of the additional initiator may provide a synergistic effect, in particular in the case of iodonium salts. Preferred iodonium salts are diphenyliodonium hexafluorophosphate, and (4-methylphenyl)[4-(2-methylpropyl)phenyl]iodonium hexafluorophosphate (Irgacure 250, commercial product available from BASF SE).

The dental compositions of the present invention may further contain particulate fillers and/ or solvents.

Suitable particulate fillers may be selected from fillers currently used in dental compositions. The filler should be finely divided and preferably has a maximum particle diameter less than about 100 μm and an average particle diameter less than about 10 μm. The filler may have a unimodal or polymodal (e.g., bimodal) particle size distribution.

The filler can be an inorganic material. It can also be a crosslinked organic material that is insoluble in the polymerizable resin, and is optionally filled with inorganic filler. The filler can be radiopaque, radiolucent or non-radiopaque. Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler may also be a filler obtainable by a process for the preparation of composite filler particles, comprising:

(a) coating a particulate filler having a median particle size (D50) of from 1 to 1200 nm with a coating composition containing a film-forming agent forming a coating layer on the surface of the particulate filler, said coating layer displaying reactive groups on the surface of the coating layer, said reactive groups being selected from addition polymerizable groups and step-growth polymerizable groups, thereby forming a coated particulate filler; subsequently or concurrently

(b) agglomerating the coated particulate filler, optionally in the presence of a further crosslinking agent and optionally in the presence of a further particulate filler not displaying reactive groups, for providing a granulation of the coated particulate filler wherein the granulation contains the coated particulate filler particles and the optional further particulate filler particles separated from and connected to each other by at least one coating layer, whereby the at least one coating layer may be crosslinked by crosslinking groups obtained by reacting the reactive groups and optionally a further crosslinking agent;

(c) optionally milling, classifying and/or sieving the granulation of the coated particulate filler; and

(d) optionally further crosslinking the granulation of the coated particulate filler; for providing composite filler particles having a median particle size (D50) of from 1 to 70 μm, wherein reactive groups are transformed into crosslinking groups obtained by reacting reactive groups and optionally a further crosslinking agent, and wherein the particulate filler is the main component by volume of the composite filler particles as further described in EP-A 2 604 247.

The dental composition of the present invention may preferably comprise 0.1 to 85 percent by weight based on the total weight of the composition of particulate filler.

Suitable solvents may be selected from water, alcohols such as methanol, ethanol, propanol (n-, i-), butanol (n-, iso-, ter.-), ketones such as acetone or the like.

The dental composition of the present invention may preferably comprise 5 to 75 percent by weight based on the total weight of the composition of a solvent.

The dental compositions of the present invention may further contain stabilizers, pigments, free radical scavengers, polymerization inhibitors, reactive and nonreactive diluents, coupling agents to enhance reactivity of fillers, rheology modifiers, and surfactants.

Suitable stabilizers may be selected from reducing agents such as vitamin C, inorganic sulfides and polysulfides and the like.

The dental composition according to the present invention may be a one-component composition.

**Examples**

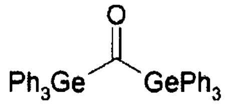

**DGK**

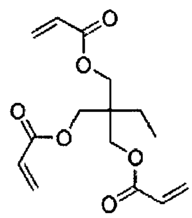

TMPTA

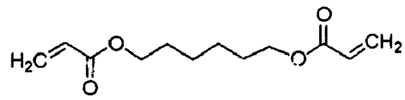

HDDA

**Scheme 1.** Chemical compounds used in this study.

***Preparation of the di(germy)ketone (DGK)***

The procedure presented in [1] for the synthesis of **DGK** (Scheme 1) was used. To a solution of triphenylgermanium hydride (1 g, 3.28 mmol) in dry THF (3 mL) at -23 °C was added n-BuLi (2.5 M in hexanes, 1.31 mL, 3.28 mmol). After 15 min. at -23 °C, ethyl formate (freshly distilled over $CaH_2$, 0.58 mL, 1.64 mmol) was added dropwise and the reaction mixture was allowed to warm at room temperature over 15 min. before being quenched with aqueous hydrochloric acid (1 M). The organic phase was separated and the aqueous phase was extracted three times with diethyl ether. The combined organic phases were dried over sodium sulfate, filtered and concentrated to give a white powder. $^1H$ NMR analysis of the crude reaction mixture indicated a 3:7 ratio of bis(triphenylgermyl)methanol /formic acid bis(triphenylgermyl)methyl ester. The latter was dissolved in dry THF (13 mL) and cooled to 0 °C. $LiAlH_4$ (0.17 g, 4.37 mmol) was then added in one portion and the stirring continued for 1 h at 0 °C before being quenched with aqueous hydrochloric acid (1 M). The organic phase was separated and the aqueous phase was extracted three times with diethyl ether. The combined organic phases were dried over sodium sulfate, filtered and concentrated to give a colorless oil. The latter was crystallized in a 9:1 EtOH/benzene mixture to give 0.63 g (60%) of bis(triphenylgermyl)methanol. $^1H$ NMR ($CDCl_3$, 300 MHz) $\delta$ 7.38-7.18 (30 H); 5.38 (s, 1H); 1,5 (1H; OH). $^{13}C$ NMR ($CDCl_3$, 75 MHz) $\delta$ 135.5; 134.0; 130.5; 129.0; 128.6; 128.1; 60.3.

To a solution of bis(triphenylgermyl)methanol (0.25 g, 3.9 mmol) in dry diethyl ether (2.3 mL) were added successively dicyclohexylcarbodiimide (0.19 g, 9.2 mmol) and pyridinium trifluoroacetate (0.09 g, 4.7 mmol). The flask was then carefully protected from light and dry DMSO (111 $\mu$L, 15.7 mmol) was added dropwise. The reaction mixture turned pink and the stirring continued overnight. The supernatant liquid was then withdrawn via syringe and filtered through a 0.2 $\mu$m syringe filter. The filtrate was then evaporated under vacuum in the absence of light to give bis(triphenylgermyl)methanone as a red solid.

**Photopolymerization procedures:**

The laminated films (25 $\mu$m thick) deposited on a $BaF_2$ pellet were irradiated with different light sources: i) polychromatic light (incident light intensity: $I_0 \approx 60$ mW $cm^{-2}$) delivered by a Xenon lamp (Hamamatsu, L8253, 150 W) and filtered to select a visible light irradiation ($\lambda$ > 400 nm) and ii) a laser diode at 473 nm (100 mW/$cm^2$).

The evolution of the double bond content was continuously followed by real time FTIR spectroscopy at about 1640 $cm^{-1}$ (Nexus 870, Nicolet) as described in [2].

Trimethylolpropane triacrylate (TMPTA) and 1,6-Hexanediol Diacrylate HDDA (Scheme 1) were selected as benchmark monomers.

**Free radical polymerization initiating ability of DGK:**

DGK can be an interesting photoinitiator upon visible light exposure i.e. quite good polymerization profiles were obtained for different benchmark acrylate monomers (TMPTA; HDDA) in presence of DGK. No polymerization was observed in absence of DGK clearly showing the role of DGK as photoinitiator for the radical polymerization (Figure 1 curve 1 vs. curve 2). DGK is also very efficient to initiate the polymerization of TMPTA upon a blue light laser diode@ 473 nm (Figure 2). In presence of iodonium salt, the polymerization profile is improved (Figure 2 curve 2 vs. curve 1).

**References:**

[1] Macromol. Rapid Commun. 2010, 31, 473-478.
[2] Fouassier, J.P.; Lalevée, J., Photoinitiators for Polymer Synthesis-Scope, Reactivity, and Efficiency. Wiley-VCH Verlag GmbH & Co. KGaA: 2012.

**Claims**

1. Photocurable dental composition comprising

(a) one or more radical-polymerizable monomers,
(b) 0.1 to 7.0 percent by weight based on the total weight of radical polymerizable monomers in the composition of a compound of the following formula (I):

$$R^1R^2R^3X(CO)X'R^4R^5R^6 \qquad (I)$$

wherein

X and X' which may be the same or different, independently represent Si or Ge,
$R^1$, $R^2$, $R^3$, $R^4$, $R^5$, and $R^6$, which may be the same or different, independently represent a hydrocarbon group having 1 to 20 carbon atoms, whereby any two hydrocarbon groups of $R^1$, $R^2$, $R^3$, $R^4$, $R^5$, and $R^6$ may be bonded to each other and form together with either X, X' or the moiety X(CO)X' to which they are attached a 3 to 12- membered heterocyclic ring,
whereby any of the hydrocarbon group or the heterocyclic ring may

- contain one or more keto groups other than the keto group of the X(CO)X' moiety, or
- be substituted by on or more groups selected from halogen atoms, a cyano group, an amino group, or a hydroxy group, and
- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group,

and

(c) a particulate filler and/or solvent.

2. The photocurable dental composition according to claim 1, which comprises less than 2.5 percent by weight based on the total weight of radical polymerizable monomers in the composition of a compound of formula (I).

3. The photocurable dental composition according to claim 1 or 2, wherein the compound of formula (I) has an absorption maximum in the range of from 400 nm to 900 nm.

4. The photocurable dental composition according to any one of the preceding claims, wherein the hydrocarbon group is an aromatic hydrocarbon group selected from a phenyl group and a naphthyl group, which groups may optionally be substituted by one to three groups selected from halogen atoms, a cyano group, an amino group, a hydroxy group, $C_{1-6}$ alkyl groups and $C_{1-6}$ alkoxy groups, or wherein the hydrocarbon group is a non-aromatic hydrocarbon group selected from a straight chain or branched alkyl group, a straight chain or branched alkenyl group, or a straight

chain or branched alkynyl group.

**5.** The photocurable dental composition according to any one of the preceding claims, wherein the radical-polymerizable monomers (a) each contain one or two radical-polymerizable groups.

**6.** The photocurable dental composition according to any one of the preceding claims, which further comprises 5 to 75 percent by weight based on the total weight of the composition of a solvent.

**7.** The photocurable dental composition according to any one of the preceding claims, which further comprises 0.1 to 85 percent by weight based on the total weight of the composition of particulate filler.

**8.** The photocurable dental composition according to any one of the preceding claims, which comprises a radical-polymerizable monomer having an acidic group.

**9.** The photocurable dental composition according to any one of the preceding claims, which is selected from a dental adhesive composition, a dental composite, a resin modified dental cement, and a pit and fissure sealant.

**10.** The photocurable dental composition according to any one of the preceding claims, which is a one-component composition.

**11.** The photocurable dental composition according to any one of the preceding claims, which contains an additional initiator which is selected from a type I initiator and a type II initiator.

**12.** The photocurable dental composition according to claim 11, wherein the additional initiator is selected from camphor quinone/amine, or iodonium salts, preferably diphenyliodonium hexafluorophosphate.

**13.** The photocurable dental composition according to any one of the preceding claims wherein the X and X' are the same.

**14.** Use of a compound of the following formula (I):

$$R^1R^2R^3Ge(CO)GeR^4R^5R^6 \qquad (I)$$

wherein

X and X' which may be the same or different, independently represent Si or Ge,
$R^1$, $R^2$, $R^3$, $R^4$, $R^5$, and $R^6$, which may be the same or different, independently represent a hydrocarbon group having 1 to 20 carbon atoms, whereby any two hydrocarbon groups of $R^1$, $R^2$, $R^3$, $R^4$, $R^5$, and $R^6$ may be bonded to each other and form together with either X or X' or with the moiety X(CO)X' to which they are attached a 3 to 12- membered heterocyclic ring,
whereby the hydrocarbon group or the heterocyclic ring may

- contain one or more keto groups other than the keto group of the moiety X(CO)X', or
- be substituted by on or more groups selected from halogen atoms, a cyano group, an amino group or a hydroxy group, and
- in case the hydrocarbon group or the heterocyclic ring contains an alkylene chain or an alkenylene chain, one or more carbon atoms in the alkylene chain or alkenylene chain may be replaced by an oxygen atom, a sulfur atom, an amide group, an ester group, a urethane group or an NH group,

for the preparation of a photocurable dental composition, preferably as defined in any of claims 1 to 13.

**Figure 1.**

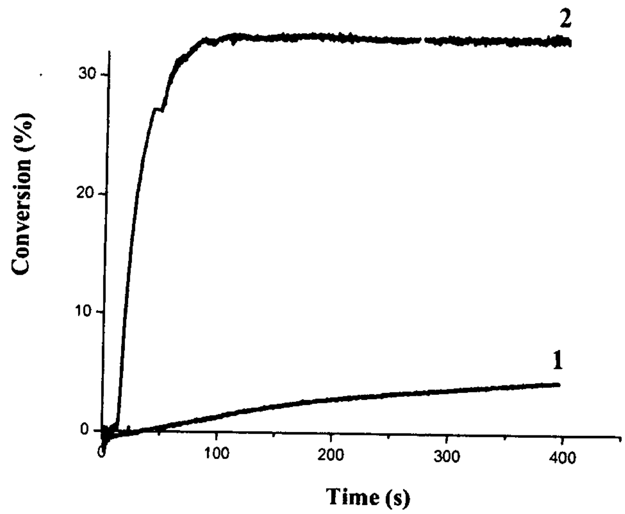


**Figure 2**

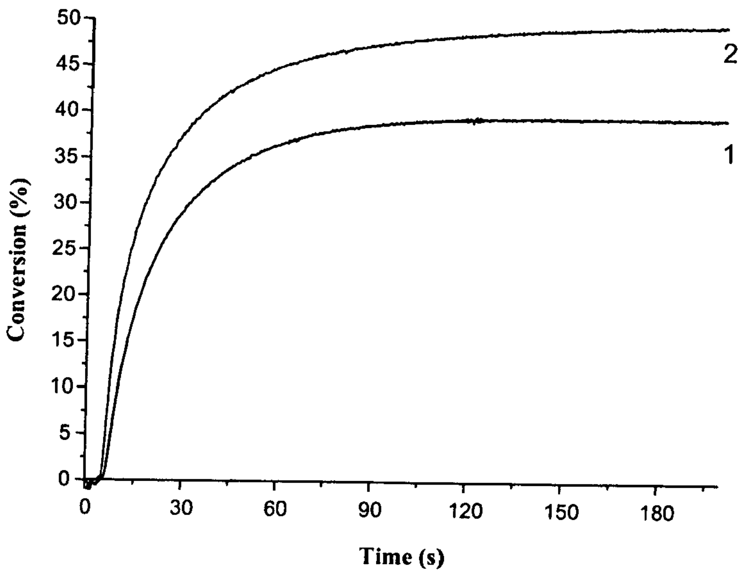

Europäisches Patentamt
European Patent Office
Office européen des brevets

# EUROPEAN SEARCH REPORT

Application Number
EP 13 00 5318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MOSZNER N ET AL: "Benzoyl germanium derivatives as novel visible light photoinitiators for dental materials", DENTAL MATERIALS, ELSEVIER, AMSTERDAM, NL, vol. 24, no. 7, 1 July 2008 (2008-07-01), pages 901-907, XP022671145, ISSN: 0109-5641, DOI: 10.1016/J.DENTAL.2007.11.004 [retrieved on 2007-12-21] * page 903, line 1 - line 3; figure 3; tables 1, 2 * ----- | 1-14 | INV. A61K6/00 |
| Y,D | M.-A. TEHFE ET AL.: MACROMOL. RAPID COMMUN., vol. 31, 2010, pages 473-478, XP002722638, * page 473 * ----- | 1-14 | |
| Y | BEATE GANSTER ET AL: "New Photocleavable Structures, 4", MACROMOLECULAR RAPID COMMUNICATIONS, vol. 29, no. 1, 2 January 2008 (2008-01-02), pages 57-62, XP055111343, ISSN: 1022-1336, DOI: 10.1002/marc.200700620 * "Dental Composites"; page 61; table 3 * ----- | 1-14 | TECHNICAL FIELDS SEARCHED (IPC)<br>A61K |

The present search report has been drawn up for all claims

1

| Place of search | Date of completion of the search | Examiner |
|---|---|---|
| Berlin | 2 April 2014 | Angiolini, Delia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2604247 A


### Non-patent literature cited in the description


- **M.-A. TEHFE et al.** *Macromol. Rapid Commun.,* 2010, vol. 31, 473-478
- **JOHANNESEN R. et al.** *J. Chem. Soc. Perkin Trans.,* 2000, vol. 1, 2677-2679
- *Macromol. Rapid Commun.,* 2010, vol. 31, 473-478
- **FOUASSIER, J.P. ; LALEVÉE, J.** Photoinitiators for Polymer Synthesis-Scope, Reactivity, and Efficiency. Wiley-VCH Verlag GmbH & Co. KGaA, 2012